(19) Europäisches Patentamt
European Patent Office
Office européen des brevets

(11) EP 1 634 581 A1

(12) **DEMANDE DE BREVET EUROPEEN**

(43) Date de publication:
**15.03.2006 Bulletin 2006/11**

(51) Int Cl.:
***A61K 8/85*** *(2006.01)* ***A61Q 1/00*** *(2006.01)*
***A61Q 19/00*** *(2006.01)*

(21) Numéro de dépôt: **05291150.0**

(22) Date de dépôt: **27.05.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **13.07.2004 FR 0451514**

(71) Demandeur: **L'OREAL**
**75008 Paris (FR)**

(72) Inventeur: **Filippi, Vanina**
**75015 Paris (FR)**

(74) Mandataire: **Chantraine, Sylvie Hélène**
**L'OREAL - D.I.P.I.**
**25-29, Quai Aulagnier**
**92600 Asnieres (FR)**

(54) **Composition cosmétique de soin ou de maquillage des matières Kératiniques comprenant un polyester et utilisation**

(57) La présente invention se rapporte à une composition cosmétique de soin ou de maquillage des matières kératiniques comprenant un milieu cosmétiquement acceptable contenant i) au moins un polyester résultant de l'estérification, par un acide polycarboxylique, d'un ester d'acide hydroxy carboxylique aliphatique comprenant au moins deux groupes hydroxyle et ii) au moins une huile volatile.

Cette composition possède des propriétés de brillance, de tenue et de confort.

EP 1 634 581 A1

## Description

La présente invention se rapporte à une composition cosmétique de maquillage ou de soin de la peau, aussi bien du visage que du corps humain, des lèvres ou des phanères, comme les cheveux, les cils, les sourcils ou les ongles, comprenant un milieu cosmétiquement acceptable contenant un polyester particulier et une huile volatile. Cette composition possède des propriétés cosmétiques remarquables et confère en particulier au maquillage ou au soin à la fois des propriétés de brillance, de tenue et de confort.

La composition de l'invention peut en particulier constituer un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

Le demandeur a trouvé de façon surprenante que l'ajout dans une composition cosmétique i) d'au moins un polyester résultant de l'estérification, par un acide polycarboxylique, d'un ester d'acide hydroxy carboxylique aliphatique, ledit ester comprenant au moins deux groupes hydroxyle à ii) au moins une huile volatile, permet d'obtenir une composition brillante et de bonne tenue, qui peut présenter en outre de bonnes propriétés de confort et/ou de non migration.

L'invention a donc pour objet une composition cosmétique, notamment de soin ou de maquillage des matières kératiniques, comprenant un milieu cosmétiquement acceptable contenant i) au moins un polyester résultant de l'estérification, par un acide polycarboxylique, d'un ester d'acide hydroxy carboxylique aliphatique, ledit ester comprenant au moins deux groupes hydroxyle et ii) au moins une huile volatile.

L'invention a également pour objet un procédé cosmétique pour conférer à un film de composition cosmétique des propriétés de brillance, de tenue et de confort, consistant à introduire dans ladite composition i) au moins un polyester résultant de l'estérification, par un acide polycarboxylique, d'un ester d'acide hydroxy carboxylique aliphatique comprenant au moins deux groupes hydroxyle et ii) au moins une huile volatile.

L'invention a encore pour objet l'utilisation de l'association i) d'au moins un polyester résultant de l'estérification, par un acide polycarboxylique, d'un ester d'acide hydroxy carboxylique aliphatique comprenant au moins deux groupes hydroxyle et ii) d'au moins une huile volatile, pour obtenir une composition cosmétique dotée de propriétés de brillance, de tenue et de confort.

L'invention a enfin pour objet l'utilisation de l'association i) d'au moins un polyester résultant de l'estérification, par un acide polycarboxylique, d'un ester d'acide hydroxy carboxylique aliphatique comprenant au moins deux groupes hydroxyle et ii) d'au moins une huile volatile, dans une composition physiologiquement acceptable, comme agents pour conférer à ladite composition de propriétés de brillance, de tenue et de confort.

### POLYESTER

Le polyester selon l'invention résulte de l'estérification

- d'un acide polycarboxylique, et
- d'un ester d'acide hydroxy carboxylique aliphatique (que l'on appellera par la suite «ester hydroxylé»).

L'ester d'acide hydroxy carboxylique aliphatique (ou ester hydroxylé) comprend au moins deux groupes hydroxyle.

L'ester hydroxylé est avantageusement issu de la réaction d'au moins un acide carboxylique aliphatique hydroxylé avec un polyol.

Ledit acide aliphatique hydroxylé comporte notamment de 2 à 40 atomes de carbone, de préférence de 10 à 34 atomes de carbone et mieux de 12 à 28 atomes de carbone ; il comporte, en outre, de 1 à 20 groupes hydroxyle, de préférence de 1 à 10 groupes hydroxyle et mieux de 1 à 6 groupes hydroxyle, susceptibles d'être estérifiés par la suite par l'acide polycarboxylique pour obtenir le polyester de la présente invention.

Ledit polyol peut comprendre de 2 à 40 atomes de carbone et mieux de 3 à 30 atomes de carbone. Le polyol est, de préférence, un polyol aliphatique. Avantageusement, le polyol n'est pas un ose.

Ledit polyol qui réagit avec l'acide hydroxylé décrit précédemment peut être partiellement ou totalement estérifié ; de façon avantageuse, le polyol est totalement estérifié.

De préférence, l'ester d'acide hydroxy carboxylique aliphatique est un ester d'acide gras hydroxylé tel que le reste acide gras comporte au moins 12 atomes de carbone, par exemple de 12 à 40 atomes de carbone, et mieux de 12 à 28 atomes de carbone.

L'ester d'acide hydroxy carboxylique aliphatique utilisable dans l'invention peut être choisi parmi :

a) les esters partiels ou totaux de monoacides carboxyliques aliphatiques mono hydroxylés linéaires saturés ;

b) les esters partiels ou totaux de monoacides carboxyliques aliphatiques mono hydroxylés insaturés tels que le

triricinoléate de glycéryle (huile de ricin) ;

c) les esters partiels ou totaux de polyol aliphatique en $C_2$ à $C_{16}$ ayant réagi avec un mono ou un polyacide carboxylique aliphatique mono ou poly hydroxylé comme notamment les triglycérides, les esters du pentaérythritol, du triméthylol propane, du propylène glycol, du néopentyl glycol, du dipentaérythritol, du polyglycérol, les esters du sorbitol ;

et leurs mélanges.

Avantageusement lorsque l'ester d'acide hydroxy carboxylique aliphatique résulte de l'estérification d'un polyacide carboxylique aliphatique tels que ceux cités ci-dessus, il ne reste pas de groupement COOH résiduel non engagé dans une liaison ester.

L'ester d'acide hydroxy carboxylique aliphatique est de préférence choisi parmi les esters de polyols aliphatiques en $C_2$ à $C_{16}$, lesdits polyols ayant réagi avec un acide gras aliphatique hydroxylé à chaîne saturée ou insaturée, comportant au moins 12 atomes de carbone. L'acide gras est de préférence l'acide ricinoléique et l'ester d'acide hydroxy carboxylique aliphatique est de préférence l'huile de ricin hydrogénée.

L'huile de ricin hydrogénée est disponible dans le commerce. Il s'agit par exemple du produit Himako P ® de Hawaken Fine Chemicals.

L'acide polycarboxylique comprend au moins deux groupes COOH. Il est avantageusement un dimère diacide d'acide(s) carboxylique(s) aliphatique(s) insaturé(s).

L'acide polycarboxylique selon l'invention est de préférence aliphatique; il est avantageusement un acide dicarboxylique aliphatique.

Selon un mode réalisation, l'acide polycarboxylique est un dimère diacide d'acide(s) gras insaturé(s), c'est-à-dire un dimère formé à partir d'au moins un acide gras insaturé, par exemple à partir d'un seul acide gras insaturé ou à partir de deux acides gras insaturés différents. L'acide gras est de préférence mono insaturé ou di insaturé. On entend par acide gras, un acide obtenu par hydrolyse de corps gras d'origine végétale ou animale.

Les dimères diacide d'acide(s) gras insaturé(s) ou encore dimère diacide sont classiquement obtenus par réaction de dimérisation intermoléculaire d'au moins un acide gras insaturé. De préférence, on dimérise un seul type d'acide gras insaturé.

Les dimères diacide d'acide(s) gras insaturé(s) sont notamment obtenus par la dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18}$.

A titre représentatif de ces acides gras insaturés, on peut notamment citer l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.

Le dimère diacide est avantageusement obtenu par dimérisation de l'acide linoléique et/ou linolénique. On utilise par exemple le produit commercial Pripol 1009 ® d'Uniqema.

Le dimère diacide est de préférence saturé, c'est-à-dire qu'il ne comporte aucune double liaison carbone carbone, et qu'il est obtenu par condensation d'acide(s) gras insaturé(s) suivie éventuellement d'une hydrogénation, pour transformer les éventuelles double liaisons en simples liaisons.

Les dimères diacide d'acide(s) gras insaturé(s) préférés sont obtenus par dimérisation de l'acide linoléique, puis éventuellement par hydrogénation du dimère ainsi obtenu. La forme hydrogénée peut être partielle ou totale et notamment correspondre à la forme saturée plus stable à l'oxydation.

On trouve également dans le commerce des dimères diacides et notamment des diacides dilinoléiques dont la stabilité vis-à-vis de l'oxydation a été améliorée par hydrogénation des doubles liaisons restant après la réaction de dimérisation.

Dans la présente invention, on peut utiliser tout dimère diacide disponible actuellement dans le commerce.

Le polyester selon l'invention a de préférence un poids moléculaire compris entre 2000 et 8000 g/mol, de préférence entre 3000 et 7000 g/mol mesuré selon la méthode détalonnage avec le polystyrène qui utilise la chromatographie par perméation de gel dans les conditions suivantes:

| | |
|---|---|
| Appareil | TOSOH SC-8010 System |
| Colonne | Shodex KF-800D + KF-805L x 2 |
| Eluant | THF |
| Temperature | Colonne isothermique 40 °C |
| Débit | 1.0 ml/min |
| Concentration | environ 0.2 poids/vol% |
| Quantité injectée | 100 µl |
| Solubilité | Dissolution complète |

Detecteur Réfractomètre Différentiel

Par exemple, le Risocast DA-L ® a une masse moléculaire en nombre comprise entre 3500 et 4000 g/mol et le Risocast DA-H ® a une masse moléculaire en nombre comprise entre 6000 et 6500 g/mol. Ces produits sont commercialisés par la société japonaise KOKYU ALCOHOL KOGYO.

Le ratio molaire entre l'acide polycarboxylique et l'ester hydroxylé utilisés pour préparer le polyester selon l'invention est de préférence compris entre 0,20 et 1, de préférence entre 0,20 et 0,40. Par exemple, ce ratio est égal à 0,75 pour le Risocast DA-H ®, et ce ratio est égal à 0,5 pour le Risocast DA-L ®.

On peut citer en particulier, comme polyester utilisable dans la composition selon l'invention :

- l'ester résultant de la réaction d'estérification de l'huile de ricin hydrogénée avec l'acide dilinoléïque dans les proportions 2 pour 1,
- l'ester résultant de la réaction d'estérification de l'huile de ricin hydrogénée avec l'acide isostéarique dans les proportions 4 pour 3.

Le polyester peut comprendre un composé de formule (1), un composé de formule (2) ou un de leurs mélanges :

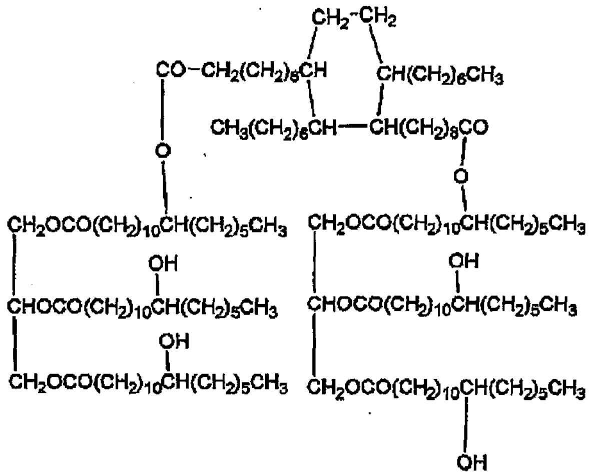

(1)

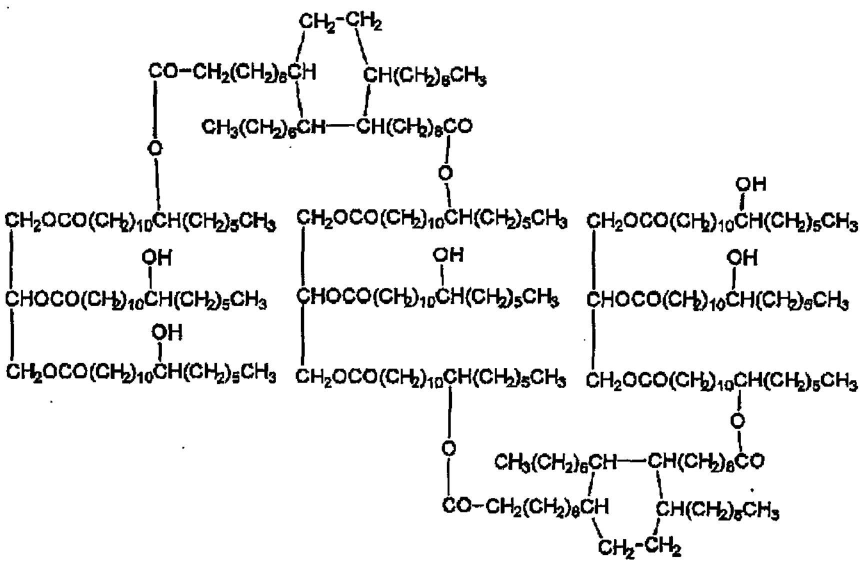

(2)

Le polyester de la présente invention est avantageusement un composé pâteux ou visqueux à température ambiante (25°C).

Par "pâteux" au sens de la présente invention, on entend désigner un polyester tel que décrit précédemment, à changement d'état solide/liquide réversible, et comportant à la température de 23°C une fraction liquide et une fraction

solide.

Le polyester a de préférence une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1 mm/s puis à une vitesse de mesure de 0,1 mm/s, la profondeur de pénétration étant de 0,3 mm. La valeur relevée de la dureté est celle du pic maximum.

Ce polyester peut en outre, à la température de 23°C, être sous la forme d'une fraction liquide et d'une fraction solide. En d'autres termes, la température de fusion commençante du polyester peut être inférieure à 23°C. La fraction liquide du polyester mesurée à 23°C peut représenter 9 à 97% en poids du composé. Cette fraction liquide à 23°C peut représenter de préférence entre 15 et 85%, de préférence encore entre 40 et 85% en poids.

La fraction liquide en poids du polyester à 23°C est égale au rapport de l'enthalpie de fusion consommée à 23°C sur l'enthalpie de fusion du polyester.

L'enthalpie de fusion du polyester est l'enthalpie consommée par le composé pour passer de l'état solide à l'état liquide. Le polyester est dit à l'état solide lorsque l'intégralité de sa masse est sous forme solide cristalline. Le polyester est dit à l'état liquide lorsque l'intégralité de sa masse est sous forme liquide.

L'enthalpie de fusion du polyester est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du polyester est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du polyester mesurée à 32°C représente de préférence de 30 à 100% en poids du composé, de préférence de 80 à 100%, de préférence encore de 90 à 100% en poids du composé. Lorsque la fraction liquide du polyester mesurée à 32°C est égale à 100%, la température de la fin de la plage de fusion du polyester est inférieure ou égale à 32°C.

La fraction liquide du polyester mesurée à 32°C est égale au rapport de l'enthalpie de fusion consommée à 32°C sur l'enthalpie de fusion du polyester. L'enthalpie de fusion consommée à 32°C est calculée de la même façon que l'enthalpie de fusion consommée à 23°C.

A titre d'exemple, le Risocast DA-L ® a une dureté à 20°C de 0,04 MPa, une fraction liquide à 23°C égale à 82 %, et une fraction liquide à 32°C égale à 90 %.

Le polyester de la composition selon la présente invention peut représenter de 1 à 99 % du poids total de la composition, de préférence de 1 à 75 %, et mieux de 5 à 60%, et mieux encore de 5 à 30%, et mieux encore de 5 à 15%.

<u>HUILE VOLATILE</u>

On peut inclure une ou plusieurs huiles volatiles dans la phase grasse de la composition

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm de Hg), et de préférence allant de 1,3 Pa à 13 000 Pa (0,01 à 100 mm de Hg), et préférentiellement allant de 1,3 Pa à 1 300 Pa (0,1 à 10 mm de Hg).

En outre, l'huile volatile a généralement un point d'ébullition, mesuré à pression atmosphérique, allant de 150°C à 260 °C, et de préférence allant de 170 °C à 250 °C.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Par huile siliconée, on entend une huile contenant au moins un atome de silicium, et notamment contenant des groupes Si-O.

Par huile fluorée, on entend une huile contenant au moins un atome de fluor.

L'huile volatile peut être siliconée ou hydrocarbonée.

L'huile volatile siliconée utilisable dans l'invention peut être choisie parmi les huiles siliconées ayant un point éclair allant de 40 °C à 102 °C, de préférence ayant un point éclair supérieur à 55 °C et inférieur ou égal à 95 °C, et préférentiellement allant de 65 °C à 95 °C.

Comme huiles siliconées volatiles utilisables dans l'invention, on peut citer les silicones linéaires ou cycliques ayant une viscosité à température ambiante inférieure à 8 cSt et ayant notamment de 2 à 7 atomes de silicium, ces

silicones comportant éventuellement des groupes alkyle ou alkoxy ayant de 1 à 10 atomes de carbone. Comme huile de silicone volatile utilisable dans l'invention, on peut citer notamment l'octaméthyl-cyclotétrasiloxane, le décaméthyl-cyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huiles siliconées volatiles utilisables dans l'invention, on peut citer les silicones décrites dans la demande FR0304259 non publiée.

L'huile volatile hydrocarbonée utilisable dans l'invention peut être choisie parmi les huiles hydrocarbonées ayant un point éclair allant de 40 °C à 102 °C, de préférence allant de 40 °C à 55 °C, et préférentiellement allant de 40 °C à 50 °C.

Comme huile volatile hydrocarbonée, on peut citer les huiles volatiles hydrocarbonées ayant de 8 à 16 atomes de carbone et leurs mélanges, et notamment les alcanes ramifiés en $C_8$-$C_{16}$ comme les iso-alcanes (appelées aussi isoparaffines) en $C_8$-$C_{16}$, l'isododécane, l'isodécane, l'isohexadécane et par exemple les huiles vendues sous les noms commerciaux d'Isopars ou de Permetyls, les esters ramifiés en $C_8$-$C_{16}$ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

L'huile volatile représente par exemple de 5 à 97,5 % du poids total de la composition, et mieux de 10 à 75 %, de préférence entre 20 et 50% du poids total de la composition.

L'huile volatile représente de préférence 20 à 50 % en poids de la composition, de préférence de 30 à 40%, de préférence 35% environ.

L'huile volatile représente de préférence 40 à 60 % de la phase grasse liquide, de préférence de 45 à 55%.

PATEUX

La composition peut également contenir un composé pâteux différent du polyester décrit précédemment.

Par "pâteux" au sens de la présente invention, on entend désigner un composé gras lipophile, à changement d'état solide/liquide réversible, et comportant à la température de 23°C une fraction liquide et une fraction solide. On entend également par « pâteux », le polylaurate de vinyle.

Le composé pâteux est avantageusement choisi parmi :

- la lanoline et ses dérivés,
- les composés fluorés polymères ou non,
- les composés siliconés polymères ou non,
- les polymères vinyliques, notamment:
    - les homopolymères d'oléfines
    - les copolymères d'oléfines
    - les homopolymères et copolymères de diènes hydrogénés
    - les oligomères linéaires ou ramifiés, homo ou copolymères de (méth)acrylates d'alkyles ayant de préférence un groupement alkyle en $C_8$-$C_{30}$
    - les oligomères h omo et copolymères d'esters vinyliques ayant d es groupements alkyles en $C_8$-$C_{30}$
    - les oligomères homo et copolymères de vinyléthers ayant des groupements alkyles en $C_8$-$C_{30}$,
    - les polyéthers liposolubles résultant de la polyéthérification entre un ou plusieurs diols en $C_2$-$C_{100}$, de préférence en $C_2$-$C_{50}$,
    - les esters,

    et leurs mélanges.

Parmi les polyéthers liposolubles, on préfère en particulier les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les pâteux esters, on préfère notamment :

- les esters d'un glycérol oligomère, notamment les esters de diglycérol, en particulier les condensats d'acide adipique et de glycérol, pour lesquels une partie des groupes hydroxyle des glycérols ont réagi avec un mélange d'acides

gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,

- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en $C_4$-$C_{50}$ et un diol ou un polyol en $C_2$-$C_{50}$, différent du polyester décrit précédemment,
- les esters aliphatiques d'ester résultant de l'estérification d'un ester d'acide hydroxycarboxylique aliphatique par un acide monocarboxylique aliphatique; et leurs mélanges, comme

  - l'ester résultant de la réaction d'estérification de l'huile de ricin hydrogénée avec l'acide isostéarique dans les proportions 1 pour 1 (1/1) ou monoisostéarate d'huile de ricin hydrogénée,
  - l'ester résultant de la réaction d'estérification de l'huile de ricin hydrogénée avec l'acide isostéarique dans les proportions 1 pour 2 (1/2) ou le diisostéarate d'huile de ricin hydrogénée,
  - l'ester résultant de la réaction d'estérification de l'huile de ricin hydrogénée avec l'acide isostéarique dans les proportions 1 pour 3 (1/3) ou triisostéarate d'huile de ricin hydrogénée,
  - et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.
Le composé pâteux représente de préférence 1 à 99%, mieux 1 à 60%, mieux 2 à 30% et mieux encore 5 à 15% en poids de la composition.

Agent dispersant hydrocarboné

Par composé « hydrocarboné », on entend un composé comportant des atomes de carbone et d'hydrogène et une ou plusieurs fonctions choisies parmi les fonctions hydroxyle, ester, éther (oxyde d'éthylène), carboxylique, amide.
L'agent dispersant hydrocarboné de l'invention est dépourvu d'atomes de fluor. Cet agent porte une ou des fonctionnalités ayant une affinité forte pour la surface des particules à disperser.
De préférence, l'agent dispersant hydrocarboné (appelé par la suite «dispersant hydrocarboné»), de la composition selon l'invention, est fluide à température ambiante (25°C), et notamment liquide et/ou présente un indice de réfraction ≥ 1,45 à 20°C (l'indice de réfraction étant mesuré au réfractomètre).
Ce dispersant hydrocarboné présente les paramètres de solubilité δd et δa selon l'espace de solubilité de Hansen satisfaisant aux conditions suivantes :
$16,2 \leq \delta_d \leq 20$ $(J/cm^3)^{1/2}$ de préférence $16,3 \leq \delta_d \leq 19$ $(J/cm^3)^{½}$, et mieux $16,9 \leq \delta_d \leq 18$ $(J/cm^3)^{1/2}$ et
$9,1 \leq \delta_a \leq 20$ $(J/cm^3)^{½}$, de préférence $10 \leq \delta_a \leq 18,1$ $(J/cm^3)^{½}$, et mieux $13 \leq \delta_a \leq 14,5$ $(J/cm^3)^{½}$
La définition des paramètres de solubilité selon HANSEN est bien connue de l'homme du métier, et notamment décrite dans l'article de C. M. HANSEN : "The three dimensional solubility parameters" J. Paint Technol. 39, 105 (1967). Ces paramètres sont aussi décrits dans le document JP-A-08-109121 de KAO et le document de D.W. Van KREVELEN "Properties of polymers" (1990), p. 190.
Selon cet espace de HANSEN :

- $\delta_d$ caractérise les forces de dispersion de LONDON issues de la formation de dipôles induits lors des chocs moléculaires ;
- δp caractérise les forces d'interactions de DEBYE entre dipôles permanents ;
- $\delta_h$ caractérise les forces d'interactions spécifiques (type liaisons hydrogène, acide/base, donneur/accepteur, etc.) ;

Les paramètres $\delta_d$, $\delta_P$ et $\delta_h$ sont généralement exprimés en $(J/cm^3)^{½}$. Ils sont déterminés à température ambiante (25°C) et en particulier selon la méthode de calcul indiquée dans le document brevet de KAO ci-dessus.
Dans la composition selon l'invention, on peut utiliser n'importe quel dispersant hydrocarboné fluide, et en particulier liquide, ou mélange de dispersants hydrocarbonés fluides satisfaisant aux relations ci-dessus. Dans ce cas, les paramètres de solubilité du mélange sont déterminés à partir de ceux des dispersants hydrocarbonés fluides pris séparément, selon les relations suivantes :

$$\delta_{Dmel} = \sum_i xi\ \delta_{Di} \ ; \qquad \delta_{pmel} = \sum_i xi\ \delta_{pi} \quad \text{et} \quad \delta_{hmel} = \sum_i xi\ \delta_{hi}$$

où xi représente la fraction volumique du dispersant hydrocarboné fluide (i) dans le mélange.

Il est à la portée de l'homme du métier de déterminer les quantités de chaque dispersant hydrocarboné fluide pour obtenir un mélange de dispersants hydrocarbonés fluides satisfaisant aux relations ci-dessus.

Avantageusement, le dispersant hydrocarboné présente une structure chimique comportant au moins un groupement polaire choisi parmi -COOH ; -OH ; oxyde d'éthylène :

- $(O\text{-}CH_2\text{-}CH_2\text{-})$ ; oxyde de propylène

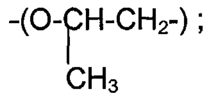

- $PO_4$; NHR ; $NR_1R_2$ avec $R_1$, $R_2$ formant éventuellement un cycle et représentant un radical alkyle ou alkoxy linéaire ou ramifié en $C_1$ à $C_{20}$ ou

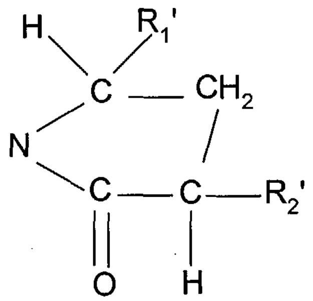

avec $R_1$' et $R_2$' qui peuvent être égal à H ou à une chaîne alkyle ou alkoxy linéaire ou ramifiée en $C_1$ à $C_{20}$.

Le dispersant hydrocarboné selon l'invention peut être choisi parmi :

- les alcools gras modifiés éther et en particulier les produits d'addition de l'oxyde d'éthylène et / ou de l'oxyde de propylène avec i) un alcool gras linéaire ou ramifié ou avec ii) un alkylphénol,
- les esters résultant de la réaction d'au moins un acide gras avec au moins un produit d'addition de l'oxyde d'éthylène et du glycérol ou avec au moins un produit d'addition de l'oxyde d'éthylène et du polyglycérol,
- les esters résultant de la réaction du glycérol ou du polyglycérol avec au moins un produit d'addition de l'oxyde d'éthylène et d'un acide gras saturé ou insaturé,
- les esters partiels résultant de la réaction d'au moins un acide gras linéaire ou ramifié, saturé ou insaturé, d'acide ricinoléique ou d'acide 12-hydroxystéarique, avec au moins un polyol tel que le glycérol, le polyglycérol, le pentaérythritol, les alcools saccharidiques tels que le sorbitol, et en particulier les esters de polyglycérol ,
- les esters résultant de la réaction du sorbitan avec au moins un acide gras linéaire ou ramifié, saturé ou insaturé,
- les esters de sorbitan modifiés éther, et en particulier les esters résultant iii) de la réaction du sorbitan avec au moins un produit d'addition de l'oxyde d'éthylène et d'un acide gras saturé ou insaturé ou iv) de la réaction d'au moins un acide gras saturé ou insaturé avec au moins un produit d'addition de l'oxyde d'éthylène et du sorbitan,
- les produits d'addition de l'oxyde d'éthylène avec l'huile de ricin et / ou l'huile de ricin hydrogénée ,
- les phosphate trialkylés et les mono, di et triphosphate alkylés,
  et leurs mélanges, ces composés satisfaisant aux paramètres de solubilité définis ci-dessus.

Le mot ester selon l'invention signifie un monoester, un diester, un triester et plus généralement un polyester.

De préférence, le dispersant hydrocarboné est choisi parmi les monoesters, les diesters et les esters résultant d'une estérification partielle, c'est à dire que l'ester final comporte une ou plusieurs fonctions -OH libre.

Avantageusement, le dispersant hydrocarboné est choisi parmi :

- les produits d'addition de 2 à 30 moles d'oxyde d'éthylène et / ou de 0 à 5 moles d'oxyde de propylène avec i) un alcool gras linéaire ou ramifié en $C_8$ à $C_{40}$, et mieux en $C_8$ à $C_{22}$ ou avec ii) un alkylphénol,
- les esters résultant de la réaction d'au moins un acide gras en $C_8$ à $C_{40}$, et mieux en $C_8$ à $C_{22}$, avec au moins un produit d'addition de 1 à 30 moles d'oxyde d'éthylène et du glycérol ou avec au moins un produit d'addition de 1 à 30 moles d'oxyde d'éthylène et du polyglycérol,
- les esters résultant de la réaction du glycérol ou du polyglycérol avec au moins un produit d'addition de 2 à 30 moles

d'oxyde d'éthylène et d'un acide gras saturé ou insaturé en $C_8$ à $C_{40}$, et mieux en $C_8$ à $C_{22}$,

- les esters partiels résultant de la réaction d'au moins un acide gras linéaire ou ramifié, saturé ou insaturé en $C_8$ à $C_{40}$ et mieux en $C_8$ à $C_{22}$, d'acide ricinoléique ou d'acide 12-hydroxystéarique, avec le glycérol, le polyglycérol, le pentaérythritol ou le sorbitol,
- les esters résultant de la réaction du sorbitan avec au moins un acide gras linéaire ou ramifié, saturé ou insaturé en $C_8$ à $C_{40}$ et mieux en $C_8$ à $C_{22}$,
- les esters résultant iii) de la réaction du sorbitan avec au moins un produit d'addition de 2 à 30 moles d'oxyde d'éthylène et d'un acide gras saturé ou insaturé en $C_8$ à $C_{40}$ et mieux en $C_8$ à $C_{22}$ ou iv) de la réaction d'au moins un acide gras saturé ou insaturé en $C_8$ à $C_{40}$ et mieux en $C_8$ à $C_{22}$, avec au moins un produit d'addition de 2 à 30 moles d'oxyde d'éthylène et du sorbitan,
- les produits d'addition de 2 à 60 moles d'oxyde d'éthylène avec l'huile de ricin et / ou l'huile de ricin hydrogénée,
- les phosphate trialkylés et les mono, di et triphosphate alkylés,
- et leurs mélanges.

De manière préférentielle, le dispersant hydrocarboné est choisi parmi :
l'alcool de myristyle oxyéthyléné à 15 groupements oxyde d'éthylène (ou OE) ($\delta_d$ = 17,33 $(J/cm^3)^{1/2}$ et $\delta_a$ = 9,28 $(J/cm^3)^{1/2}$), le monoisostéarate de polyglycérol-2 oxyéthyléné à 5 OE ($\delta_d$ = 17,34 $(J/cm^3)^{1/2}$ et $\delta_a$ = 12,22 $(J/cm^3)^{1/2}$), le diisostéarate de polyglycérol-3 ($\delta_d$ = 16,96 $(J/cm^3)^{1/2}$ et $\delta_a$ = 10,4 $(J/cm^3)^{1/2}$), le monoisostéarate de glycérol ($\delta_d$ = 16,32 $(J/cm^3)^{½}$ et $\delta_a$ = 11,01 $(J/cm^3)^{1/2}$), le monoisostéarate de polyglycérol-2 ($\delta_d$ = 17,03 $(J/cm^3)^{1/2}$ et $\delta_a$ = 13,25 $(J/cm^3)^{1/2}$), l'isostéarate de polyglycérol-3 ($\delta_d$ = 17,38 $(J/cm^3)^{1/2}$ et $\delta_a$ = 14,48 $(J/cm^3)^{1/2}$), l'isostéarate de polyglycérol-4 ($\delta_d$ = 17,57 $(J/cm^3)^{1/2}$ et $\delta_a$ = 15,37 $(J/cm^3)^{½}$), le monoisostéarate de polyglycérol-6 ($\delta_d$ = 17,86 $(J/cm^3)^{1/2}$ et $\delta_a$ = 16,61 $(J/cm^3)^{1/2}$), le monoisostéarate de polyglycérol-10 ($\delta_d$ = 18,22 $(J/cm^3)^{½}$ et $\delta_a$ = 18,41 $(J/cm^3)^{1/2}$), le monooléate de polyglycérol-2 ($\delta_d$ = 17,14 $(J/cm^3)^{½}$ et $\delta_a$ = 13,39 $(J/cm^3)^{1/2}$), l'isostéarate de sorbitan ($\delta_d$= 17,33 $(J/cm^3)^{½}$ et $\delta_a$ = 13,56 $(J/cm^3)^{½}$), le monooléate de sorbitan ($\delta_d$ = 17,32 $(J/cm^3)^{1/2}$ et $\delta_a$ = 13,66 $(J/cm^3)^{½}$), le monooléate de sorbitan oxyéthyléné à 5 OE ($\delta_d$ = 17,56 $(J/cm^3)^{1/2}$ et $\delta_a$= 12,47 $(J/cm^3)^{1/2}$), et leurs mélanges.

Avantageusement, le dispersant hydrocarboné est choisi parmi les esters partiels de polyglycérol et d'acide isostéarique, les esters partiels de polyglycérol et d'acide oléique, les esters partiels de sorbitan et d'acide oléique, et leurs mélanges.

Comme dispersant hydrocarboné utilisable préférentiellement dans la composition selon l'invention, on peut choisir le monoisostéarate de polyglycérol-2 tel que le Salacos 41 fabriqué ou commercialisé par la société Nisshin Oil Mills, le diisostéarate de polyglycérol-3 tel que le Lameform TGI fabriqué ou commercialisé par la société Cognis, le monooléate de polyglycérol-2 tel que le Rylo PG 29 fabriqué ou commercialisé par la société Danisco Ingredients, le monooléate de sorbitan tel que le Span 80 fabriqué ou commercialisé par la société Uniqema, et leurs mélanges.

Les quantités des différents ingrédients de la composition selon l'invention seront données en pourcentages en poids par rapport au poids total de la composition.

L'agent dispersant hydrocarboné peut représenter de 0,5 à 40 %, de préférence de 3 à 20% et mieux de 5 à 15% du poids total de la composition.

<u>COLORANT</u>

Avantageusement, la composition de l'invention peut comprendre, en outre, au moins une matière colorante qui peut être choisie parmi les colorants solubles ou dispersibles dans la composition, les pigments, les nacres et leurs mélanges. Les colorants sont de préférence des colorants liposolubles, bien que les colorants hydrosolubles puissent être utilisés. Cette matière colorante peut représenter de 0,001 à 98 %, de préférence de 0,5 à 85% et mieux de 1 à 60 % du poids total de la composition.

Pour une composition sous forme de pâte ou coulée telle que les rouges à lèvres ou les produits de maquillage du corps, on utilise en général de 0,5 à 50% de matière colorante, de préférence de 2 à 40 % et mieux de 5 à 30%, par rapport au poids total de la composition.

Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

De préférence, la composition de l'invention, comprend une phase particulaire avantageusement colorée pouvant représenter de 0,001 à 50 % du poids total de la composition, de préférence de 0,01 à 40 % et mieux de 0,05 à 30 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles

dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 30 % (si présents) du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % (si présentes) du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

La composition contient avantageusement des pigments goniochromatiques, par exemple des pigments multicouches interférentiels, et/ou des pigments réfléchissants. Ces deux types de pigments sont décrits dans la demande FR0209246 dont le contenu est incorporé par référence dans la présente demande.

CHARGES

Les charges peuvent être présentes à raison de 0,001 à 35 % du poids total de la composition, de préférence 0,5 à 15 %.

On peut notamment citer

- le talc, le mica, le kaolin, l'amidon
- les poudres de Nylon® (Orgasol notamment)
- les poudres de polyéthylène,
- les poudres de polytétrafluoroéthylène (Téflon®),
- le nitrure de bore,
- des microsphères de copolymères telles que l'Expancel® (Nobel Industrie),
- le Polytrap® 603 (Dow Corning),
- le Polypore® L 200 (Chemdal Corporation)
- les microbilles de résine de silicone (Tospearl ® de Toshiba, par exemple),
- les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass
- les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI.

La charge peut être par exemple une charge dont la granulométrie moyenne est inférieure à 100 μm, notamment comprise entre 1 et 50 μm, par exemple entre 4 et 20 μm.

La charge peut être de toute forme, essentiellement sphérique ou sous la forme de plaquettes.

CIRE

La composition peut contenir, en outre, au moins une cire. Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.
La composition selon l'invention contient avantageusement de la cire de polyéthylène de masse moléculaire en poids comprise entre 300 et 700, notamment égale à 500 g/mol.
A titre indicatif, la cire peut représenter de 0,01 à 50 %, de préférence de 2 à 40 %, et mieux de 5 à 30 % du poids total de la composition.

<u>HUILE NON VOLATILE</u>

La composition peut également comprendre au moins une huile non volatile. L'huile non volatile peut être choisie parmi:

- les huiles hydrocarbonées d'origine animale telles que le perhydrosqualène ;
- les huiles hydrocarbonées végétales telles que les triglycérides liquides d'acides gras ayant de 4 à 10 atomes de carbone comme les triglycérides des acides heptanoïque ou octanoïque ou l'huile de jojoba ;
- les hydrocarbures linéaires ou ramifiés, d'origine minérale ou synthétique tels que les huiles de paraffine et leurs dérivés, la vaseline ;
- les alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones (telles que la phényl triméthicone vendue sous le nom commercial DC556 par Dow Corning), les phényl diméthicones, les phényl triméthylsiloxy diphényl siloxanes, les diphényl diméthicones, les diphényl méthyldiphényl trisiloxanes, les 2-phényl éthyl triméthyl-siloxysilicates ;
- les acides gras ayant de 12 à 26 atomes de carbone comme l'acide oléïque
- les esters non volatiles comprenant moins de 40 atomes de carbone
- les esters et polyesters de dimère diol, tels que les esters de dimère diol et d'acide gras, et les esters de dimère diols et de diacide.

  Les esters de dimère diol et d'acide mono-carboxylique peuvent être obtenus à partir d'acide mono-carboxylique comprenant de 4 à 34 atomes de carbone, notamment de 10 à 32 atomes de carbone, lesquels acides sont linéaires, ramifiés, saturés ou insaturés.

  A titre illustratif des exemples d'acide mono-carboxylique convenant à l'invention, on peut notamment citer les acides gras.

  Les esters de dimère diol et d'acide dicarboxylique peuvent être obtenus à partir d'un dimère diacide dérivé en particulier de la dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18}$.

  Selon une variante particulière, il s'agit plus particulièrement du dimère diacide dont dérive également le dimère diol à estérifier.

  Les esters de dimère diol peuvent être obtenus à partir d'un dimère diol produit par hydrogénation catalytique d'un dimère diacide tel que décrit précédemment, par exemple le diacide dilinoléique hydrogéné.

  A titre illustratif des esters de dimère diol, on peut notamment citer les esters de diacides dilinoléiques et de dimères diols dilinoléiques commercialisés par la société NIPPON FINE CHEMICAL sous la dénomination commerciale LUSPLAN DD-DA5® et DD-DA7® .
- et leurs mélanges.

On utilise de préférence des huiles non volatiles d'origine végétale ou synthétique.
Les huiles non volatiles peuvent représenter de 0,001 à 90 % du poids total de la composition, de préférence de 0,05 à 60 % et mieux de 1 à 35 %.

ADDITIFS

La composition de l'invention peut comprendre, en outre, tout additif complémentaire usuellement utilisé dans le domaine concerné, tel que de l'eau, des antioxydants, des conservateurs, des neutralisants, des gélifiants lipophiles ou des composés non aqueux liquides, des gélifiants de phase aqueuse, des dispersants, des actifs cosmétiques. Ces additifs, à l'exception de l'eau qui peut représenter de 0 à 70 % et par exemple de 1 à 50 et mieux de 1 à 10 % du poids total de la composition, peuvent être présents dans la composition à raison de 0,0005 à 20% du poids total de la composition et mieux de 0,001 à 10%.

Comme actif cosmétique utilisable dans l'invention, on peut citer les vitamines A, E, C, $B_3$, F, les provitamines comme le D-panthénol, la glycérine, les actifs apaisants comme l'$\alpha$-bisabolol, l'aloe vera, l'allantoïne, les extraits de plantes ou les huiles essentielles, les agents protecteurs ou restructurants comme les céramides, les actifs "fraîcheur" comme le menthol et ses dérivés, les émollients (beurre de cacao, diméthicone), les hydratants (arginine PCA), les actifs antirides, les acides gras essentiels, les filtres solaires, et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

GALENIQUES

Les applications des compositions selon l'invention sont multiples et concernent l'ensemble des produits cosmétiques colorés ou non et plus particulièrement les rouges à lèvres.

La composition de l'invention peut se présenter sous la forme de composition solide, compactée ou coulée notamment en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

Elle peut se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. De préférence, elle se présente sous forme de composition à phase continue huileuse et notamment anhydre ; dans ce cas, elle peut contenir une phase aqueuse à un taux inférieur à 5 %.

La composition selon l'invention peut se présenter sous la forme d'une composition, colorée ou non, de soin de la peau, sous forme d'une composition de protection solaire ou de démaquillage ou encore sous forme d'une composition hygiénique. Si elle contient des actifs cosmétiques, elle peut alors être utilisée comme base de soin ou de traitement non thérapeutique pour la peau comme les mains ou le visage ou pour les lèvres (baumes à lèvres, protégeant les lèvres du froid et/ou du soleil et/ou du vent), produit de bronzage artificiel de la peau.

La composition de l'invention peut également se présenter sous la forme d'un produit de maquillage coloré de la peau, en particulier du visage comme un blush, un fard à joues ou à paupières, de maquillage du corps comme un produit de tatouage semi-permanent ou de maquillage des lèvres comme un rouge ou un brillant à lèvres, présentant éventuellement des propriétés de soin ou de traitement non thérapeutique, un produit de maquillage des phanères comme par exemple un vernis à ongles, un mascara, un eyeliner, un produit de coloration ou de soin des cheveux.

De préférence, la composition selon l'invention se présente sous forme d'un rouge à lèvres ou d'un brillant à lèvres.

Bien entendu la composition de l'invention doit être physiologiquement acceptable (en particulier cosmétiquement acceptable), à savoir non toxique et susceptible d'être appliquée sur la peau, les phanères ou les lèvres d'êtres humains.

Par « cosmétiquement acceptable », on entend agréable de goût, de toucher, d'aspect et/ou d'odeur, applicable plusieurs jours pendant plusieurs mois.

La composition selon l'invention peut être fabriquée par les procédés connus, généralement utilisés dans le domaine cosmétique.

Les exemples qui suivent ont pour but d'illustrer de manière non limitative l'objet de la présente invention. Les quantités sont données en pourcentage massique.

**Exemple 1: Rouge à lèvres en stick**

| Matières premières | Pourcentage en poids |
|---|---|
| | |

Suite de tableau

| Matières premières | Pourcentage en poids |
|---|---|
| Polylaurate de vinyle | 6,5 |
| Polyester d'acide dimère dilinoléïque et d'huile de ricin hydrogénée (Risocast DAL®, société KOKYU ALCOHOL KOGYO) | 9,2 |
| PVP/eicosène | 7,4 |
| Cire microcristalline | 3,9 |
| Cire ozokérite | 2,7 |
| N-lauroyl L-lysine | 2,0 |
| Kaolin | 2,0 |
| Conservateurs | 0,2 |
| Polyisobutylène hydrogéné | 17,0 |
| Phényltrimethicone commercialisé ou fabriqué par la société Dow Corning sous la référence DC 556 | 17,0 |
| OLEATE DE SORBITANE SPAN 80 V de ICI-UNIQEMA | 2,8 |
| Pigments | qs |
| Charges | 4,0 |

**Exemple 2: Rouge à lèvres en stick**

| Matières premières | Pourcentage en poids |
|---|---|
| Cire ozokérite | 2 |
| OXYDE DE FER BRUN (CI: 77491) | 4,0 |
| Cire microcristalline | 3 |
| OLEATE DE SORBITANE SPAN 80 V de ICI-UNIQEMA | 7,2 |
| POLYLAURATE DE VINYLE | 9,7 |
| COPOLYMERE ACETATE DE VINYLE / STEARATE D'ALLYLE (65/35) MEXOMERE PQ (P 2724 de CHIMEX | 1 |
| MONO/DI/TRI-BEHENATE DE GLYCERYLE COMPRITOL 888 ATO de GATTEFOSSE | 3 |
| PHENYL TRIMETHYLSILOXY TRISILOXANE (VISCOSITE: 20 CST DC 556) | 2,8 |
| COPOLYMERE DI-METHACRYLATE D'ETHYLENE GLYCOUMETHACRYLATE DE LAURYLE POLYTRAP 603 ULTRA-LITE SORPTION POLYMER POWDER de LAMBERT-RIVIERE | 1 |
| ISO-HEXADECANE | 17 |
| LAQUE D'ALUMINIUM DE BLEU BRILLANT FCF SUR ALUMINE (12/88) (CI: 42090:2 + 77002) | 0,1 |
| SEL DE CALCIUM DU ROUGE LITHOL B | 0,1 |
| MALATE DE DI-ISO-STEARYLE | 14,4 |
| POLY PHENYLTRIMETHYLSILOXY DIMETHYLSILOXANE (VISCOSITE: 1000 CST Belsil 1000) | 7,6 |
| OXYDE DE TITANE RUTILE TRAITE ALUMINE/SILICE/TRIMETHYOLPROPANE (CI: 77891) | 2,2 |

Suite de tableau

| Matières premières | Pourcentage en poids |
|---|---|
| COPOLYMERE DIMERES DILINOLEYL DIOL/DIMERES DILINOLEIQUES LUSPLAN DD-DA7 de NIPPON FINE CHEMICAL | 15 |
| Polyester d'acide dimère dilinoléïque et d'huile de ricin hydrogénée (Risocast DA-L®, société KOKYU ALCOHOL KOGYO) | 9,3 |
| Conservateur | qs |
| Total | 100 |

## Revendications

1. Composition cosmétique comprenant i) au moins un polyester résultant de l'estérification, par un acide polycarboxylique, d'un ester d'acide hydroxy carboxylique aliphatique, ledit ester comprenant au moins deux groupes hydroxyle et ii) au moins une huile volatile.

2. Composition selon la revendication précédente, **caractérisée en ce que** l'acide polycarboxylique est un acide polycarboxylique aliphatique, de préférence un acide dicarboxylique aliphatique.

3. Composition selon la revendication précédente, **caractérisée en ce que** l'acide polycarboxylique est un dimère diacide formé à partir d'au moins un acide gras insaturé.

4. Composition selon la revendication précédente **caractérisée en ce que** le dimère est un dimère d'un acide gras insaturé en $C_8$ à $C_{34}$, notamment en $C_{12}$ à $C_{22}$ en particulier en $C_{16}$ à $C_{20}$ et plus particulièrement en $C_{18}$.

5. Composition selon la revendication précédente, **caractérisée en ce que** le ou les acides gras insaturés sont choisis parmi l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.

6. Composition selon la revendication 5, **caractérisée en ce que** l'acide gras insaturé est l'acide linoléique, et que le dimère est l'acide dilinoléïque.

7. Composition selon l'une quelconque des revendications précédentes, **caractérisée en ce que** le dimère diacide est saturé, c'est-à-dire qu'il ne comporte aucune double liaison carbone carbone, et qu'il est obtenu par condensation d'acide(s) gras insaturé(s) suivie éventuellement d'une hydrogénation, pour transformer les éventuelles double liaisons en simples liaisons.

8. Composition selon l'une des revendications précédentes, **caractérisée en ce que** l'ester d'acide hydroxy carboxylique aliphatique est choisi parmi :

   a) les esters partiels ou totaux de monoacides carboxyliques aliphatiques mono hydroxylés linéaires saturés ;
   b) les esters partiels ou totaux de monoacides carboxyliques aliphatiques mono hydroxylés insaturés ;
   c) les esters partiels ou totaux de polyols aliphatiques en $C_2$ à $C_{16}$ ayant réagi avec un mono ou un poly acide carboxylique aliphatique mono ou poly hydroxylé ;
   et leurs mélanges.

9. Composition selon l'une des revendications précédentes, **caractérisée en ce que** l'ester d'acide hydroxy carboxylique aliphatique est choisi parmi les esters de polyols aliphatiques en $C_2$ à $C_{16}$, lesdits polyols ayant réagi avec un acide gras aliphatique hydroxylé à chaîne saturée ou insaturée, comportant au moins 12 atomes de carbone.

10. Composition selon l'une des revendications précédentes, **caractérisée en ce que** l'ester d'acide hydroxy carboxylique aliphatique est choisi parmi les esters saturés ou insaturés, de polyols aliphatiques en $C_2$ à $C_{16}$, lesdits polyols ayant réagi avec l'acide ricinoléique.

**11.** Composition selon l'une des revendications précédentes, **caractérisée en ce que** l'ester d'acide hydroxy carboxylique aliphatique est l'huile de ricin hydrogénée.

**12.** Composition selon l'une des revendications précédentes, **caractérisée en ce que** le polyester présente une fraction liquide et une fraction solide à 23°C et une dureté allant de 0,001 à 0,5 MPa, de préférence de 0,002 à 0,4 MPa.

**13.** Composition selon l'une des revendications précédentes, **caractérisée en ce que** le polyester représente de 1 à 99%, de préférence de 1 à 75 %, et mieux de 5 à 60% du poids total de la composition.

**14.** Composition selon l'une des revendications précédentes, **caractérisée en ce que** l'huile volatile est choisie parmi

- citer les silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl-cyclotétrasiloxane, le décaméthyl-cyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane,
- les huiles volatiles hydrocarbonées ayant de 8 à 16 atomes de carbone et leurs mélanges, et notamment les alcanes ramifiés en $C_8$-$C_{16}$ comme les iso-alcanes (appelées aussi isoparaffines) en $C_8$-$C_{16}$, l'isododécane, l'isodécane, l'isohexadécane et par exemple les huiles vendues sous les noms commerciaux d'Isopars ou de Permetyls, les esters ramifiés en $C_8$-$C_{16}$ comme le néopentanoate d'iso-hexyle, et
- leurs mélanges.

**15.** Composition selon l'une des revendications précédentes, **caractérisée en ce que** l'huile volatile représente 20 à 50 % , de préférence 30 à 40% en poids de la composition.

**16.** Composition selon l'une des revendications précédentes, dans laquelle l'huile volatile représente 40 à 60 %, de préférence de 45 à 55% en poids de la phase grasse liquide.

**17.** Composition selon l'une des revendications précédentes, **caractérisée en ce qu'**elle se présente sous forme d'un rouge à lèvres ou d'un brillant à lèvres, d'un fard à joues ou à paupières, d'un mascara, d'un eye-liner, d'un vernis à ongles, d'un produit de bronzage artificiel de la peau, d'un produit de coloration ou de soin des cheveux.

**18.** Composition selon l'une des revendications précédentes, **caractérisée en ce qu'**elle comprend, en outre, au moins une matière colorante.

**19.** Composition selon l'une des revendications précédentes, **caractérisée en ce qu'**elle comprend, en outre, au moins un additif complémentaire choisi parmi l'eau, les cires, les composés pâteux, les charges, les antioxydants, les conservateurs, les neutralisants, les gélifiants lipophiles ou de composés non aqueux liquides, les gélifiants de phase aqueuse, les dispersants, les actifs cosmétiques et leurs mélanges.

**20.** Composition selon l'une des revendications précédentes, **caractérisée en ce qu'**elle se présente sous forme coulée ou compactée.

**21.** Composition selon l'une des revendications précédentes, **caractérisée en ce qu'**elle se présente sous forme anhydre.

**22.** Composition selon l'une des revendications précédentes, **caractérisée en ce qu'**elle se présente sous forme d'un rouge à lèvres ou d'un brillant à lèvres.

**23.** Procédé cosmétique pour conférer à un film de composition cosmétique des propriétés de brillance, de tenue et de confort, consistant à introduire dans ladite composition i) au moins un polyester de l'estérification, par un acide polycarboxylique, d'un ester d'acide hydroxylé carboxylique aliphatique comprenant au moins deux groupes hydroxyle et ii) au moins une huile volatile.

**24.** Utilisation de l'association i) d'au moins un polyester résultant de l'estérification, par un acide polycarboxylique, d'un ester d'acide hydroxy carboxylique aliphatique comprenant au moins deux groupes hydroxyle et ii) d'au moins une huile volatile, pour obtenir une composition brillante, de bonne tenue et confortable.

**25.** Utilisation de l'association i) d'au moins un polyester résultant de l'estérification, par un acide polycarboxylique aliphatique, d'un ester d'acide hydroxy carboxylique comprenant au moins deux groupes hydroxyle aliphatique et ii) d'au moins une huile volatile, dans une composition physiologiquement acceptable, comme agent pour conférer à ladite composition des propriétés de brillance, de tenue et de confort.

Office européen des brevets

# RAPPORT PARTIEL DE RECHERCHE EUROPEENNE

qui selon la règle 45 de la Convention sur le brevet européen est consideré, aux fins de la procédure ultérieure, comme le rapport de la recherche européenne

Numéro de la demande: EP 05 29 1150

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 2004/028640 A1 (ARNAUD PASCAL ET AL) 12 février 2004 (2004-02-12)<br>* le document en entier *<br>----- | 1-25 | A61K7/02<br>A61K7/06<br>A61K7/04 |
| X | FR 2 795 309 A (NIPPON FINE CHEMICAL COMPANY LIMITED) 29 décembre 2000 (2000-12-29)<br>* le document en entier *<br>----- | 1-25 | |
| X | US 6 670 441 B1 (O'LENICK, JR. ANTHONY J ET AL) 30 décembre 2003 (2003-12-30)<br>* le document en entier *<br>----- | 1-25 | |
| X | US 6 342 527 B1 (O'LENICK, JR. ANTHONY J ET AL) 29 janvier 2002 (2002-01-29)<br>* le document en entier *<br>----- | 1-25 | |
| X | US 2003/077234 A1 (ARNAUD PASCAL) 24 avril 2003 (2003-04-24)<br>* le document en entier *<br>-----<br>-/-- | 1-25 | |

DOMAINES TECHNIQUES RECHERCHES (Int.Cl.7)

A61K

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

1

| Lieu de la recherche | Date d'achèvement de la recherche | Examinateur |
|---|---|---|
| Munich | 25 octobre 2005 | Yon, J-M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C08)

Office européen des brevets

# RAPPORT PARTIEL DE RECHERCHE EUROPEENNE

Numero de la demande
EP 05 29 1150

| DOCUMENTS CONSIDERES COMME PERTINENTS | | | CLASSEMENT DE LA DEMANDE (Int.Cl.7) |
|---|---|---|---|
| Catégorie | Citation du document avec indication, en cas de besoin, des parties pertinentes | Revendication concernée | |
| X | PATENT ABSTRACTS OF JAPAN vol. 2002, no. 09, 4 septembre 2002 (2002-09-04) & JP 2002 128629 A (KOSE CORP), 9 mai 2002 (2002-05-09) * abrégé * ----- | 1-25 | |
| X | PATENT ABSTRACTS OF JAPAN vol. 2003, no. 12, 5 décembre 2003 (2003-12-05) & JP 2004 107355 A (KOKYU ALCOHOL KOGYO CO LTD), 8 avril 2004 (2004-04-08) * abrégé * ----- | 1-25 | DOMAINES TECHNIQUES RECHERCHES (Int.Cl.7) |

1

EPO FORM 1503 03.82 (P04C11)

Office européen des brevets

**RECHERCHE INCOMPLETE**
**FEUILLE SUPPLEMENTAIRE C**

Numéro de la demande
EP 05 29 1150

Revendications ayant fait l'objet de recherches incomplètes:
1-25

Revendications n'ayant pas fait l'objet de recherches:
-

Raison pour la limitation de la recherche:

Les présentes revendications ,23,24 et 25 ont trait à un nombre très important de
composés. Un fondement et exposé au sens de l'article
84 et 83 CBE ne peut cependant être trouvé que pour un nombre très restreint de ces
composés revendiqués.La non-conformité
avec les exigences quant au fond est telle qu'une recherche significative portant sur
l'ensemble de l'objet revendiqué n'a pas pu être effectuée (règle 45 CBE et Directives
B-VIII, 3.). L'étendue de la recherche a par conséquent été limitée aux parties des revendications qui présentent un fondement et un exposé, c'est à dire les parties ayant trait aux composés en pages 4 et 5 de la description ainsi que le polyester exemplifié.

**ANNEXE AU RAPPORT DE RECHERCHE EUROPEENNE RELATIF A LA DEMANDE DE BREVET EUROPEEN NO.** EP 05 29 1150

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-10-2005

| Document brevet cité au rapport de recherche | Date de publication | Membre(s) de la famille de brevet(s) | Date de publication |
|---|---|---|---|
| US 2004028640 A1 | 12-02-2004 | CN 1477949 A | 25-02-2004 |
| | | EP 1429718 A1 | 23-06-2004 |
| | | FR 2829924 A1 | 28-03-2003 |
| | | WO 03035024 A1 | 01-05-2003 |
| | | JP 2005510496 T | 21-04-2005 |
| FR 2795309 A | 29-12-2000 | AUCUN | |
| US 6670441 B1 | 30-12-2003 | AUCUN | |
| US 6342527 B1 | 29-01-2002 | AUCUN | |
| US 2003077234 A1 | 24-04-2003 | EP 1254649 A1 | 06-11-2002 |
| | | FR 2824266 A1 | 08-11-2002 |
| | | JP 2002332223 A | 22-11-2002 |
| JP 2002128629 A | 09-05-2002 | AUCUN | |
| JP 2004107355 A | 08-04-2004 | AUCUN | |

EPO FORM P0460

Pour tout renseignement concernant cette annexe : voir Journal Officiel de l'Office européen des brevets, No.12/82